# EUROPEAN PATENT APPLICATION

(11) **EP 2 770 049 A1**
(43) Date of publication of application: **27.08.2014**
(21) Application number: 12841149.3
(22) Date of filing: 17.10.2012
(51) Int. Cl.: C12N 1/12, C12N 1/13, C12M 1/00, C12M 1/04, C12M 1/42

(54) **BIOMASS PRODUCTION METHOD AND APPARATUS USED IN SAID METHOD**

(30) Priority: 21.10.2011 ES; 12.06.2012 ES
(71) Applicant: Normacon 21 S.L., 08028 Barcelona (ES)
(72) Inventor: HERMS GAVALDÁ, Antonio, E-08028 Barcelona (ES)
(74) Representative: Morgades y Manonelles, Juan Antonio
(86) International application number: PCT/ES2012/070718
(87) International publication number: WO 2013/057348

(57) **Abstract**

The invention relates to a method for accelerating biomass production, based on the culture of microalgae, using, for implementing said method, a reactor specifically designed therefor, in which a culture of water with microalgae, with nutrients and CO2 is placed in said reactor, a direct current being passed through the interior thereof.

The invention also relates to a second, variant embodiment comprising the addition of means for oxygenating the microalgal culture that is the subject of the method, involving the further incorporation of a closed circuit for utilizing the water used by said apparatus, plus the corresponding filter.

## Description

### Object of the Invention.

More specifically the invention relates to a method for accelerating biomass production, based on the culture of microalgae, using, for implementing said method, a reactor specifically designed therefor, in which a culture of water with microalgae, with nutrients and CO2 is placed in said reactor, a direct current being passed through the interior thereof.

Another object of the invention is a second variant embodiment comprising the addition of means for oxygenating the microalgal culture that is the subject of the method, involving the further incorporation of a closed circuit for utilizing the water used by said apparatus, plus the corresponding filter.

### State of the Art.

As a result of the exhaustion of the current energy model based on crude oil and its derivatives, a number of new processes have arisen to obtain energy from other raw materials, preferably renewable, which in addition to energy alternatives, also aim to minimise environmental impact so that its use and consumption will not affect the ecosystem as a consequence of waste generated.

An example of the above is wind energy, along with solar energy, in its thermal, thermo electrical and photoelectrical variants, which have experienced continuous and promising growth that will lead to new sources of energy. Another example of alternative energy is based on the cultivation of microalgae to produce and obtain biofuels, due to its innumerable advantages which is in many aspects complementary to the rest of renewable energies, as it addresses one of the aspects that are more difficult to solve with other technologies; A sustainable and economic fuel for transport.

Microalgae are organisms that live in water, either freshwater or saltwater in very humid environments. Microalgae in nature are highly important, as thanks to photosynthesis, they are able to transform inorganic material into organic material using solar energy, which is stored in its biological structures and is later used by other creatures that feed off them.

In the solar photosynthesis process, atmospheric CO2 combines with water and as a result produces oxygen that releases sugars into the atmosphere, which the microalgae will use to produce different substances such as cellulose, which configures its structure, oils and others.

Microalgae have a very high rate of multiplication, and they are able to absorb and store a large amount of solar energy. There are three ways to grow algae: in ponds and pools in the open air, in greenhouses and finally in photobioreactors.

From the industrial point of view, cultivation in photobioreactors is the most popular due to its productivity, and consists of transparent conduits isolated from the outside, in which the microalgae grow. These tubes are placed outdoors to capture the solar radiation better. A computer-controlled system supplies CO2 and nutrients to the microalgae to optimize productivity.

Therefore, the cultivation of microalgae presents a series of important advantages compared with the use of other agricultural products currently used; Firstly, they have much higher productivity than any other type of organic material, in second place, they do not emit CO2 to the atmosphere, in third place, the production of biofuel from microalgae has no effect on the food market, and finally, it does not use wood from trees and therefore does not harm the forests.

The most important products derived from cultivation of microalgae to produce energy are biodiesel, bioethanol, biomass, and bio-petroleum.

### Scope of the Invention.

The aim of the invention is to provide a novel method for the industrial production of biomass without using practically any energy and without affecting the environment.

Another aim of the invention is to design a bioreactor used in the procedure, especially designed for its operation.

A last aim of the invention is the incorporation of to the equipment used in the biomass-production method of a system for making use of the water used by the bioreactor.

### Description of the Invention.

One of the objects of the invention is a method to increase the multiplication rate of the algae, in fresh water, with the contribution of CO2, nutrients, and the application of a low-voltage electric field. The application of a low-voltage electric field, according to the method object of the invention, has been revealed as a factor, which notably increases the multiplication of the microalgae and consequently the volume of biomass generated.

Another object of the invention is the design of a new type of bioreactor which is physically suitable for the implementation of said method, formed by two covers, from each of which emerges perpendicularly an opening, between the covers and surrounding the mouths a transparent polyethylene cover for making use of the solar energy or similar material, secured to the mouth by the corresponding clamps.

In one of the covers is the CO2 gas inlet by means the corresponding check valve, the water inlet with a shut-off valve, the inlet for nutrients with a shut-off valve and a level gauge for controlling the water inside the bioreactor.

Inside the bioreactor there is a spiral-shaped cathode, with input and electrical direct current connection through one of the covers and communicated to the outside, there is a ventilation tube, which may optionally be fitted with an extractor fan to avoid the interior temperature of the bioreactor exceeding an acceptable value, and therefore killing the microalgae.

The direct current voltage circulating through the cathode is between 0.5 and 1.5 volts.

Optionally, the bioreactor may work in series with other bioreactors, by connecting a bioreactor with the next, through a single conduit with a cut-off valve.

Another of the aims of the present invention is a bioreactor which, as indicated in claim 5, comprises between two covers with an opening present in one of its surfaces, an interior volume delimited by a transparent material cover, the ends of which span the openings by means of the corresponding clamps, in the inside of which there is a cathode connected to a direct current source, one of the covers having the water inlet and outlet, the nutrient inlet, the CO2 gas inlet, and the control of the interior level of the liquid present in the body of the reactor with the corresponding level gauge.

The implementation of the aforementioned bioreactor, as well later investigations, has revealed the advantage of a breathing system directly focused on the cultivation of the microalgae carried out inside the bioreactor, with means external to it.

Therefore, one of the objects of the invention, is the modification of the elements complementary to the bioreactor, so it can work in a closed circuit, making use of the water which is used, among other things, for growing said microalgae, for which an electric pump and a filter is incorporated, connected to the inlet and outlet of the body of the bioreactor.

Another of the aims of the invention is the inclusion of a Venturi in this closed circuit, which enables air from the outside to be taken and added to the water circulating in the closed circuit in a programmed manner, and an air vent outlet with the corresponding chimney on the body of the bioreactor, with which its interior temperature will be maintained at suitable values.

The closed circuit makes use of the corresponding electronic control box already existing in the purpose of the main patent introducing a CPU to govern all the elements, both interior and exterior, of the bioreactor, with the essential and main feature that the air inlet through the Venturi will be discontinuous, so that the growth of the microalgae will not be affected as a consequence of continuous air injection, for which purpose the electric pump controlled by the CPU will produce an intermittent current of recirculation water in combination with the Venturi, all the elements of the bioreactor such as water inlets and outlets, CO2 inlet, valves and fan directly connected to the corresponding CPU box by means of their respective cables.

Other details and characteristics shall be shown throughout the description below referring to drawings attached to this report which are shown for illustrative but not limiting purposes only in a drawing of the invention.

### Description of the drawings.

Below is a list of the different parts of the invention, that are indicated in the above drawings with their respective numbers; (10, 10') bioreactor, (11,12) covers of (10), (13,14) covers of (22), (15) transparent cover, (16) cathode, (17) tube, (18) fan, (19) solar panel, (20) CO2 inlet, (21) H2O inlet, (22) second bioreactor, (23) H2O outlet, (24) interior volume of bioreactor (10), (25) interior volume, (26) level control, (27) nutrients inlet, (28) opening, (29) conduit, (30) cut-off valve, (31) water recirculation conduit, (32) electric pump, (33) filter, (34) Venturi, (35) CPU, (36) cables, (37) air vent outlet, (38) chimney.
Figure 1 is a front elevation view of a first embodiment of the bioreactor (10), showing one of its covers (11), being arranged on (11) the CO2 gas inlet (20), the H2O inlet (21), the nutrients inlet (27), the H2O outlet (23) and the level gauge (26).
Figure 2 is a top plan view of the bioreactor (10) of figure 1, working in series with a second bioreactor (22), joined by a conduit (29) and a cut-off valve (30).
Figure 3 is a side elevation view of a cover (13) of the bioreactor (10) of figure 1, this cover (13) having a cylindrical opening (28).
Figure 4 is a side elevation view of the bioreactor (10) of the previous figures, but with improvements, which include an electric pump (32), a filter (33), a Venturi (34), a CPU (35), and some cables (36) for bidirectional connection of the aforementioned components with the CPU (35), which will also include the corresponding computer, forming an electronic control box.

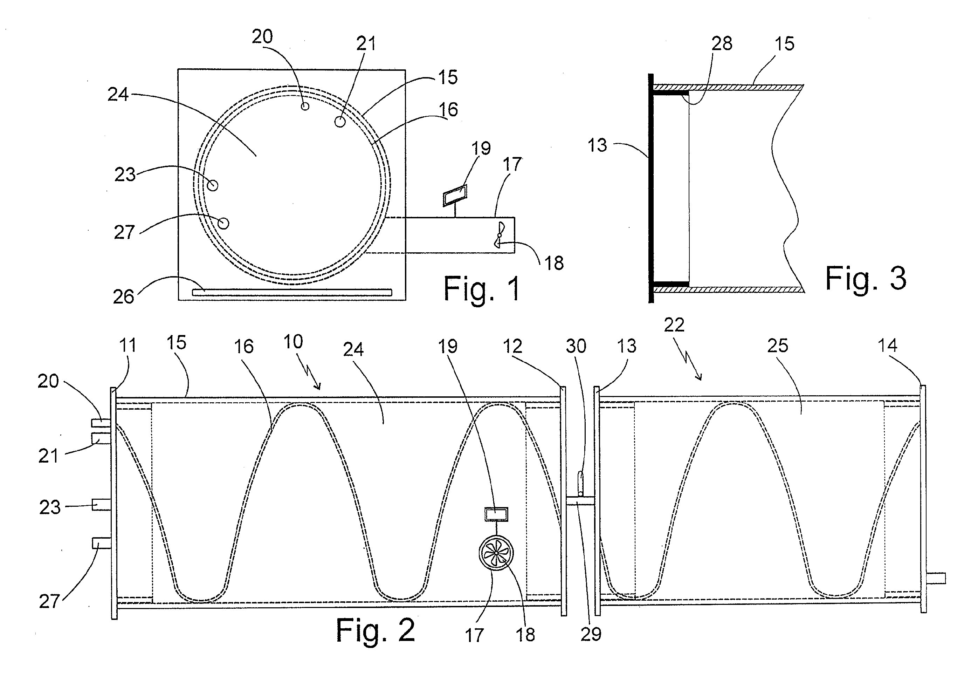

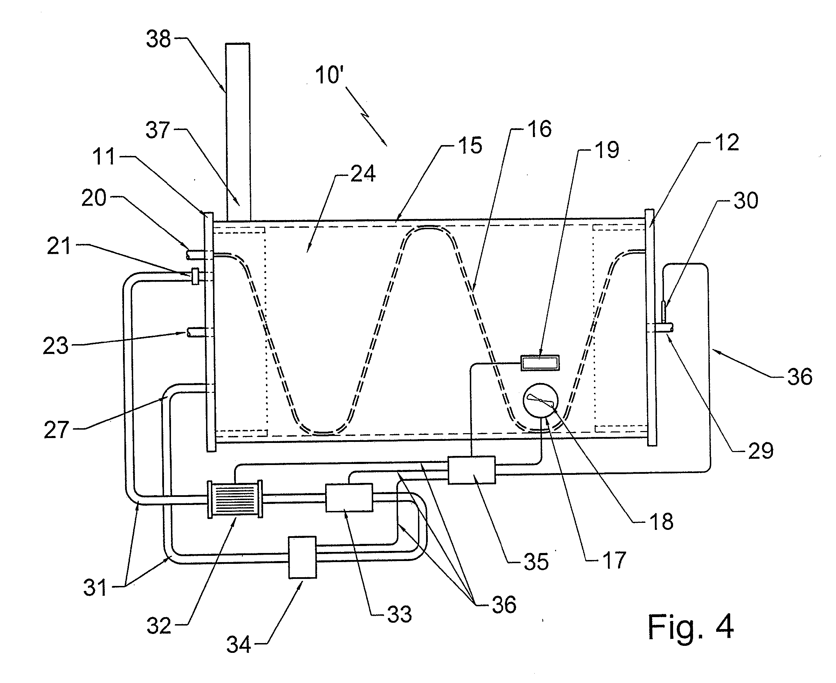

To facilitate the interpretation of the invention the numbering of the different parts of the embodiment of the bioreactor in figure 4, these references are identical to those of the first embodiment of the bioreactor of figures 1 to 3.

### Description of the Invention.

In one of the preferred embodiments, as may be seen in figure 1, a first embodiment of the bioreactor (10) according to the invention comprises a body formed by covers (11-12), the openings (28) of which are surrounded at the ends with a cover of polyethylene or similar transparent material that allows the passage of sunlight (15), in the interior volume (25) of (10), there is a spiral-shaped cathode (16), although other configurations are possible. Thanks to the spiral-shaped configuration, the cathode (16) also supports the bioreactor (10) cover (15).

The material chosen for the cathode (16) is stainless steel, although it could be manufactured in iron wire, although this has displayed some drawbacks, such as the rust generated, which could end up killing the microalgae, therefore stainless steel has been chosen as more suitable for the passage of current.

One of the covers, for instance (13), has some machined orifices for the inlet (20) of CO2 gas, therefore this inlet (10) will be fitted with the corresponding check valve (not shown in the figures), the same cover (13) also contains the water inlet (21) with a cut-off valve (not shown in the figures) and finally a outlet (23) for H2O, for emptying the bioreactor (10), nutrients can be added to the bioreactor using the water inlet and its cut-off valve. To control the level of liquid inside the bioreactor (10), the cover (11) also has a level gauge (26).

The multiplication of microalgae by photosynthesis requires control of the interior temperature of the bioreactor, for which it is designed so that the interior volume (25) can be ventilated by means of a ventilation tube (17) and inside there is an electric fan (18), supplied with electricity by a solar panel (19), as can be seen in figure 1.

The method, according to one of the aims of the invention comprises at least the following operations:
- Filling the bioreactor (10) with water and micro-algae.
- Supplying nutrients to the interior of the bioreactor (10).
- Supplying CO2 to the interior of the bioreactor (10).
- Creation of an electric direct current field by means of the cathode (16), powered by a direct current supply.

The operational procedure of the bioreactor (10) consists in filling (10) with water and microalgae by the inlet (21), which can be automatic, using remotely controlled electro valves, as well as emptying, using the outlet (23). In addition, the CO2 and nutrients inlet (20) and (27) may be automated and programmed with the corresponding electronics, without changing the essence of the method.

Alternatively the procedure may be carried out industrially by connecting in series several bioreactors (10), joining them by means of a conduit (29) and a cut-off valves (30), as can be seen in figure 2, and automating the assembly by means of electronics to control the respective inlets and outlets of water, inputs of nutrients, inputs of CO2 gas, needing practically no energy, as this may be obtained from solar panels such as (19).

In the second of the preferred embodiments of this invention, as can be seen in figure 4, the bioreactor (10') incorporates some conduits (31) that connect the water inlet (21) with the water outlet (23), thus making use of the water employed in feeding the bioreactor (10'), proceeding to have inserted in series in said conduits (31), a filter (33) and an electric pump (32) which enables a continuous current of water through these conduits (31) and through the interior of the bioreactor (10').

In addition, with the aim of oxygenating the mixture of water, nutrients, CO2 and microalgae discontinuously, air is introduced and consequently oxygen, using a Venturi (34) fitted in series in the conduits (31), the body of the bioreactor (10') having an air vent outlet (37) and a chimney (38).

By experiments carried out, the operation of the Venturi (34) must be programmed in times controlled by CPU (35), which will vary according to the nature of the microalgae used and their concentration.

Additionally, as described in the first embodiment of the invention, all rest of the internal and external organs of the bioreactor (10') will be connected to the CPU (35) by means of cables (36), as it is not necessary for the electric pump (32) or the fan (38) to operate continuously, therefore the energy consumed by the bioreactor (10') is minimum, as the small amount of power needed by the electric pump (32) and the fan (18) will be supplied by solar energy from a panel (19), these improvements produce a 300% increase in performance compared with the bioreactor (10) described in figures 1 to 3.

Consequently, the biomass-production method and apparatus used in said method of figures 1 to 3 is enlarged with a new operation consisting in discontinuous circulation in closed water circuit.

Having sufficiently described this invention using the figures attached, it is easy to understand that any modification may be made to the detail which may be deemed to be appropriate, whenever these changes do not alter the essence of the invention summarised in the following claims.

## Claims

1. **"BIOMASS-PRODUCTION METHOD"** using microalgae, which multiply by photosynthesis from sunlight in an aqueous medium such as water, **characterised in that** the method comprises at least the following operations:
- Filling the bioreactor with water and micro-algae.
- Supplying nutrients to the interior of the bioreactor.
- Supplying CO2 to the interior of the bioreactor.
- Creation of an electric direct current field by means of a cathode, powered by a direct current supply.

2. **"BIOMASS-PRODUCTION METHOD"** according to the claim 1, **characterised in that** the direct current voltage circulating through the cathode is between 0.5 and 1.5 volts.

3. **"BIOMASS-PRODUCTION METHOD"** according to the claim 1, **characterised in that** the opening and closing of water, nutrients and CO2 gas inlets and outlets, are governed by an electronic control box.

4. **"BIOMASS-PRODUCTION METHOD"** according to the claim 2, **characterised in that** the cathode is spiral shaped and it is placed under the transparent material cover of the bioreactor.

5. **"BIOMASS-PRODUCTION METHOD"** according to the claim 1, **characterised in that** the method comprises, in addition to the aforementioned operations, the closed circuit circulation of water with filtration and addition of air.

6. **"APPARATUS USED IN THE BIOMASS-PRODUCTION METHOD"** according to any of claims 1 to 5, **characterised in that** the body of the bioreactor comprises, between two covers with an opening present in one of its surfaces, an interior volume delimited by a transparent material cover, the ends of which span the openings by means of the corresponding clamps, in the inside of which there is a cathode connected to a direct current source, one of the covers having the water inlet and outlet, the nutrient inlet, the CO2 gas inlet, and the control of the interior level of the liquid present in the body of the reactor with the corresponding level gauge.

7. **"APPARATUS USED IN THE BIOMASS-PRODUCTION METHOD"** according to the previous claim, **characterised in that** the body of the bioreactor incorporates a ventilation tube with an electric fan in its upper part, powered by a solar energy panel.

8. **"APPARATUS USED IN THE BIOMASS-PRODUCTION METHOD"** according to the claim 6, **characterised in that**, alternatively, the apparatus used industrially comprises the connection in series of several bioreactors, joining them by means of a conduit and a cut-off valves, and automating the assembly by means of electronics to control the respective inlets and outlets of water, inputs of nutrients, inputs of CO2 gas, needing practically no energy, as this may be obtained from solar panels.

9. **"APPARATUS USED IN THE BIOMASS-PRODUCTION METHOD"** according to any of claims 6 to 8, **characterised in that**, additionally, the bioreactor has, as an external element, some conduits between the water inlet and outlet, between which there is an electric pump for circulating water, a Venturi filter for injecting air into the water, a CPU controlling the bioreactor and its external element and on the body of the bioreactor an air vent outlet with its corresponding chimney.

10. **"APPARATUS USED IN THE BIOMASS-PRODUCTION METHOD"** according to the claim 9, **characterised in that** the operation of the electric pump is discontinuous and the operation times are controlled by orders given by the CPU.

11. **"APPARATUS USED IN THE BIOMASS-PRODUCTION METHOD"** according to the claim 9, **characterised in that** the type of operation of the bioreactor is discontinuous and the Venturi operation times are controlled by orders given by the CPU.
